# NOUVEAU FASCICULE DE BREVET EUROPEEN

(11) **EP 1 843 667 B2**
(45) Date de publication et mention de la décision concernant l'opposition: **26.10.2022**
(45) Mention de la délivrance du brevet: 25.05.2016
(21) Numéro de dépôt: 06709242.9
(22) Date de dépôt: 03.02.2006
(51) Int. Cl.: A23K 20/111, A23K 10/30, A23K 10/37, A23K 50/10

(54) **ADDITIF ALIMENTAIRE POUR RUMINANTS A BASE D'EUGENOL ET DE CINNAMALDEHYDE**
NAHRUNGSMITTELZUSATZSTOFF FÜR WIEDERKÄUER AUF EUGENOL- UND ZIMTSÄUREALDEHYDBASIS
FOOD ADDITIVE FOR RUMINANTS BASED ON EUGENOL AND CINNAMALDEHYDE

(30) Priorité: 03.02.2005 FR 0501489
(43) Date de publication de la demande: 17.10.2007
(73) Titulaire: PANCOSMA FRANCE S.A.S., 01200 Bellegarde-sur-Valserine (FR); Archer-Daniels-Midland Company, Decatur, IL 62526 (US)
(72) Inventeur: GAUTIER, François, F-74600 Seynod (FR); KAMEL, Christopher, E-43892 Miami Playa (ES); CALSAMIGLIA, Sergio, E-08190 San Cugat Del Valles (ES); DOANE, Perry, Decatur, Indiana 46733 (US)
(74) Mandataire: Cabinet Poncet
(86) Numéro de dépôt international: PCT/FR2006/000253
(87) Numéro de publication internationale: WO 2006/082326

(56) Documents cités:
- EP-A- 0 630 577
- EP-A- 1 266 578
- EP-A1- 1 419 811
- WO-A-02/085132
- WO-A-03/094628
- CARDOSO P W; CALSAMIGLIA S; FERRET A; KAMEL C: "Effects of natural plant extracts on ruminal protein degradation and fermentation profiles in continuous culture" JOURNAL OF ANIMAL SCIENCE, vol. 82, no. 11, octobre 2004 (2004-10), pages 3230-3236, XP002379395
- BELITZ HD; GROSCH W: "Food Chemistry" 1999, SPRINGER VERLAG , BERLIN , XP002379398 page 908; tableau 22.3
- BULLERMAN L B; LIEU F Y; SEIER S A: "INHIBITION OF GROWTH AND AFLA TOXIN PRODUCTION BY CINNAMON AND CLOVE OILS CINNAMIC ALDEHYDE AND EUGENOL" JOURNAL OF FOOD SCIENCE, vol. 42, no. 4, 1977, pages 1107-1109, XP008063756 US
- PESSOA L M ET AL: "Antihelminthic activity of essential oil of Ocimum gratissimum Linn. and eugenol against Haemonchus contortus" VETERINARY PARASITOLOGY, ELSEVIER SCIENCE, AMSTERDAM, NL, vol. 109, 2002, pages 59-63, XP002309503 ISSN: 0304-4017

## Description

### DOMAINE TECHNIQUE DE L'INVENTION

La présente invention concerne les additifs alimentaires utilisés dans la nutrition animale ou la médecine vétérinaire pour améliorer le bien-être et les performances zootechniques des animaux ruminants, par exemple la production de viande, la production laitière.

On connaît déjà l'effet favorable d'additifs alimentaires à base d'antibiotiques.

Mais les antibiotiques ont des effets secondaires indésirables, pouvant notamment conduire à une accoutumance des germes, et provoquant une administration involontaire et inacceptable d'antibiotiques aux consommateurs.

On a par ailleurs développé des additifs de substitution à base d'extraits de plantes. Par exemple, le document JP 11196776 enseigne d'améliorer la qualité, la fraîcheur, la capacité de conservation de la viande bovine par administration aux bovins, dans leur ration alimentaire, d'un additif combinant de la vitamine E avec un extrait de plante choisi parmi un groupe comprenant le poivre noir, le poivre blanc, les graines de céleri, le coriandre, le cumin, le clou de girofle, l'origan, la sauge, le thym, le laurier, la cannelle, etc.

Le document WO 02/085132 A1 enseigne d'améliorer l'efficacité d'utilisation des aliments par les animaux ruminants en ajoutant dans la ration alimentaire un additif à base d'un surfactant non ionique et d'un agent anti-oxydant. L'additif modifie la fermentation dans le rumen, en favorisant la production d'acide propyonique. Le document décrit un exemple dans lequel l'additif alimentaire peut contenir en outre, parmi d'autres substances similaires, de l'eugenol et du cinnamaldehyde en tant que palatants ou substances destinées à modifier le goût.

Le document SU 1460783 décrit l'utilisation d'une combinaison d'eugenol et de cinnamaldehyde comme agent aromatisant imitant l'arôme du cola, pour un usage dans l'industrie alimentaire.

Le document WO 2004/091307 A2 décrit des additifs alimentaires destinés à protéger certains animaux terrestres ou aquatiques contre les maladies. Il n'y a pas d'exemple d'application aux animaux ruminants. L'eugenol et le cinnamaldehyde sont cités parmi un grand nombre de composants possibles de l'additif.

Le brevet GB 2 118 420 A décrit des additifs alimentaires pour améliorer la production de viande de volaille. Le cinnamaldehyde et l'eugenol sont cités parmi les nombreuses substances pouvant entrer dans la composition de l'additif. L'effet obtenu est d'améliorer le goût de la viande obtenue grâce aux qualités aromatiques des substances utilisées.

Le document EP 0 630 577 A1 enseigne d'améliorer la digestibilité des aliments destinés aux animaux ruminants, en ajoutant des additifs alimentaires pouvant contenir de l'eugenol. Le cinnamaldehyde n'est pas cité.

Le document Cardozo et al., Journal of Animal Science, 82, p. 3230-3236 (2004), décrit l'utilisation d'huile essentielle de cannelle pour augmenter la protéolyse des aliments par les ruminants.

Le document EP 1 266 578 décrit l'utilisation d'une préparation contenant du cinnamaldehyde pour la fabrication d'une composition destinée au traitement curatif ou préventif des pathologies animales au cours desquelles on observe une présence surnuméraire de germes anaérobies. Le cinnamaldehyde peut être issu d'une huile essentielle de cannelle.

### EXPOSE DE L'INVENTION

Le problème proposé par l'invention est d'améliorer encore de façon sensible les performances zootechniques des ruminants, notamment la production de viande et/ou la production laitière, en utilisant d'autres moyens et sans utiliser d'antibiotiques.

La présente invention résulte de l'observation surprenante d'un effet positif de synergie entre un dérivé aromatique phénolique, plus particulièrement l'eugenol, en combinaison avec un dérivé aromatique aldéhydique, plus précisément le cinnamaldehyde, la combinaison des deux molécules permettant d'améliorer la digestibilité des aliments pour animaux ruminants, et donc la performance zootechnique des animaux ruminants.

### DESCRIPTION DES MODES DE REALISATION PREFERES

Ainsi, selon un premier aspect, l'invention propose d'améliorer la digestibilité des aliments par les animaux ruminants en utilisant un mélange actif contenant de l'eugenol et du cinnamaldehyde.

Par ruminants, on entend les espèces vaches laitières, bovins, ovins, brebis, chèvres.

Par eugenol, on entend le composé naturel identique obtenu par synthèse, ou le composé naturel provenant préférentiellement des plantes du genre Eugenia telles que Eugenia caryophyllata, Eugenia aromatica et Eugenia polyantha ou des plantes du genre Syzygium telles que Syzygium aromaticum et Syzygium polyanthum.

Par cinnamaldehyde, on entend le composé naturel identique obtenu par synthèse, ou le composé naturel provenant préférentiellement des plantes du genre Cinnamomum telles que Cinnamomum burmannii, Cinnamomum cassia, Cinnamomum camphora, Cinnamomum loureirii, Cinnamomum tamala, Cinnamomum osmophloeum, Cinnamomum porrectum, et Cinnamomum verum.

Pour un bon effet de synergie, le rapport entre les quantités respectives en poids d'eugenol et de cinnamaldehyde présentes dans le mélange est de préférence compris entre 1 et 5, plus avantageusement compris entre 1 et 1,8 environ, l'eugenol étant alors prépondérant.

Le mélange eugenol-cinnamaldehyde peut avantageusement être incorporé à la ration alimentaire de l'animal.

De préférence, le mélange d'eugenol et de cinnamaldehyde est présent dans la ration alimentaire du ruminant selon une proportion de 1 à 20 ppm (grammes par tonne) environ par rapport à la matière sèche de la ration, ce qui correspond à une quantité journalière de 20 à 400 mg environ pour une ration alimentaire journalière de 20 kg de matière sèche.

Selon l'invention, le mélange eugenol-cinnamaldehyde peut être utilisé directement sous forme liquide.

Toutefois, le mélange sera préférentiellement utilisé sous la forme de poudre après adsorption ou absorption sur un support solide particulaire. L'opération d'adsorption ou d'absorption peut comprendre une étape de chauffage raisonnable, sans inconvénient sur l'efficacité du mélange actif.

Les supports pouvant être utilisés pour l'adsorption ou l'absorption du mélange selon l'invention peuvent être ceux employés classiquement dans les domaines pharmaceutique et alimentaire, tels que notamment : les silices, les celluloses, les sels, les carbonates de calcium, les alginates, les sciures, les gommes, les graisses hydrogénées.

La mise sur support solide de l'eugenol et du cinnamaldehyde est de préférence réalisée en proportion d'au moins 5 % en poids, plus avantageusement d'au moins 20 % en poids de l'ensemble support solide-eugenol-cinnamaldehyde.

Selon un autre aspect, l'invention propose un procédé pour augmenter l'efficacité de l'utilisation des aliments par un animal ruminant, comprenant le fait d'ajouter à la ration alimentaire de l'animal un mélange d'eugenol et de cinnamaldehyde en quantité appropriée pour augmenter la digestibilité de la ration alimentaire, caractérisé en ce que le rapport entre les quantités respectives en poids d'eugenol et de cinnamaldehyde est compris entre 1 et 5.

Le mélange d'eugenol et de cinnamaldehyde peut avantageusement être adsorbé ou absorbé sur un support solide particulaire, et ensuite le support solide particulaire peut être ajouté à la ration alimentaire.

Selon un autre aspect, l'invention propose un additif alimentaire pour animaux ruminants, contenant, dans un excipient alimentaire, un mélange d'eugenol et de cinnamaldehyde adsorbé ou absorbé sur un support solide particulaire, caractérisé en ce que le rapport entre les quantités respectives en poids d'eugenol et de cinnamaldehyde est compris entre 1 et 5.

De préférence, le mélange est présent en proportion comprise entre 0,00005 % et 5 % en poids de l'additif alimentaire.

Dans l'additif alimentaire, l'excipient peut par exemple contenir, en poids, de 0,1 % à 1 % d'un mélange de vitamines, de 20 % à 80 % de sels minéraux, de 20 % à 80 % de protéines, de 20 % à 80 % de produits issus de meunerie.

Selon un autre aspect, l'invention propose une ration alimentaire à grande digestibilité pour animaux ruminants, contenant un mélange d'eugenol et de cinnamaldehyde en proportion de 1 à 20 ppm environ par rapport à la matière sèche de la ration alimentaire, caractérisé en ce que le rapport entre les quantités respectives en poids d'eugenol et de cinnamaldehyde est compris entre 1 et 5.

Une ration alimentaire pour animaux ruminants peut notamment contenir le mélange d'eugenol et de cinnamaldehyde avec :
- au moins 50 % de céréales,
- au moins 25 % de tourteaux d'oléagineux,
- au moins 20 % de produits issus de meunerie.

De préférence, dans le mélange d'eugenol et de cinnamaldehyde présent dans la ration alimentaire, le rapport entre les quantités respectives en poids d'eugenol et de cinnamaldehyde sera compris entre 0,2 et 5, plus avantageusement compris entre 1 et 1,8 environ.

Des essais ont été réalisés afin de démontrer l'efficacité du mélange eugenol-cinnamaldehyde selon l'invention, et son utilisation bénéfique pour améliorer les propriétés zootechniques des animaux ruminants. Les cinq études ci-après démontrent cette efficacité.

### Etude 1

Au cours de cette première étude, on a analysé les effets d'un mélange selon l'invention sur la digestibilité de la ration alimentaire des animaux ruminants, par une étude effectuée "in vitro" selon la méthode de Tilley et Terry (1963).

Pour une description plus complète de cette méthode, on pourra se référer à la publication : Tilley, J.M.A. and Terry, R.A. 1963. A two stage technique for the in vitro digestion of forage crops. J. Brit. Grassland Soc. 18 - 104-111.

On a utilisé, pour l'incubation :
a) la ration de base composée de 100 % de luzerne déshydratée, supplémentée par une quantité égale soit de l'eugenol, soit du cinnamaldehyde, soit d'un mélange des deux composants ;
b) le tampon de MacDougall ;
c) le liquide du rumen prélevé à jeun de quatre animaux équipés en permanence
d'une canule au niveau du rumen et nourris avec la ration de base.

En pratique, on a prévu quatre récipients de 250 millilitres chacun contenant du liquide du rumen et du tampon de MacDougall dans la proportion de 1 à 4, auquel on a ajouté 1 gramme (matière sèche) de la luzerne, contenu dans un sachet en Dacron^{™} perméable. Le liquide ruminal était originaire de vaches fistulées dans les premiers 100 jours de lactation. Le premier récipient a été conservé en l'état, sans ajout. Dans le second récipient, on a ajouté 30 ppm (parties par million en poids) d'eugenol. Dans le troisième récipient, on a ajouté 30 ppm de cinnamaldehyde. Dans le quatrième récipient, on a ajouté 15 ppm d'eugenol et 15 ppm de cinnamaldehyde.

Les récipients ont été incubés à 39°C pendant 24 heures.

Les sachets en Dacron^{™} ont été prélevés, lavés et séchés à 55°C. Différents paramètres de digestibilité ont été déterminés selon la méthode de Van Soest et al. (1991). A cet égard, pour une description plus complète de la méthode, on pourra se référer à la publication suivante : Van Soest, P.J. Robertson, J.D., and Lewis, B.A. 1991. Methods for dietary fibre, neutral detergent fibre and non-starch polysaccharides in relation to animal nutrition. J. Dairy Sc. 74: 3583-3597.

Ainsi, la digestibilité, déterminée par la disparition de matière, a été mesurée en évaluant la digestibilité de la matière sèche (DMd), la digestibilité de la fibre digestible neutre (NDFd) et la digestibilité de la fibre digestible acide (ADFd) ; les mesures ont été faites à l'aide d'un appareil ANKOM 200.

Les résultats obtenus sont résumés dans le tableau 1 ci-dessous :

| Traitement | Contrôle négatif (sans additif) | Cinnamaldehyde | Eugenol | Eugenol + Cinnamaldehyde |
|---|---|---|---|---|
| DMd (%) | 37.77 | 39.06 | 40.02 | 40.46 |
| NDFd (%) | 5.60 | 4.83 | 5.63 | 10.08 |
| ADFd (%) | 9.03 | 8.53 | 8.05 | 11.47 |

Le tableau montre que la combinaison de l'eugenol et du cinnamaldehyde selon l'invention améliore notamment la digestibilité des fibres de la luzerne déshydratée, qui constitue une matière première "clé" de l'alimentation de la vache laitière. Par contre, l'eugenol seul ou le cinnamaldehyde seul n'améliorent pas la digestibilité des fibres.

### Etude 2

On a testé, dans cette seconde étude, l'effet d'une variation des proportions relatives d'eugenol et de cinnamaldehyde dans un additif alimentaire, pour la production de viande et la production laitière.

Dans cet essai, seize vaches laitières de la race Holstein-Freisan, dans leur huitième semaine de lactation, ont été partagées pour un essai de type Latin Carré 4 × 4. Chaque période de traitement a duré deux semaines.

Un premier groupe a reçu une alimentation dépourvue d'additif.

Un second groupe a reçu une alimentation contenant 30 ppm de cinnamaldehyde.

Un troisième groupe a reçu une alimentation contenant un mélange de 30 ppm de cinnamaldehyde et 15 ppm d'eugenol.

Un quatrième groupe a reçu une alimentation contenant un mélange de 30 ppm d'eugenol et 15 ppm de cinnamaldehyde.

Au cours de chaque période de traitement, on a mesuré la consommation d'aliments et la production laitière.

Les résultats sont contenus dans le tableau 2 ci-dessous :

| | Traitement | | | |
|---|---|---|---|---|
| | Control négatif (sans additif) | Cinnamaldehyde 30 | Cinnamaldehyde 30 + Eugenol 15 | Eugenol 30 + Cinnamaldehyde 15 |
| Consommation d'aliment (kg MS/animal/j) | 3.087a | 2.571b | 2.632b | 3.287a |
| Production laitière (1/animal/j) | 37.28 | 37.23 | 35.76 | 38.35 |

On constate qu'un additif alimentaire à base de cinnamaldehyde seul, ou un additif alimentaire à base d'un mélange de cinnamaldehyde et d'eugenol dans lequel le cinnamaldehyde est nettement prépondérant, conduisent à une réduction de la consommation d'aliments (en kilogramme de matière sèche par animal et par jour), et conduisent à une réduction de la production laitière (en litre par animal et par jour).

Par contre, le traitement utilisant un additif alimentaire dans lequel l'eugenol est prépondérant dans un mélange d'eugenol et de cinnamaldehyde conduit à une augmentation de la consommation d'aliments et de la production laitière.

### Etude 3

Cette troisième étude a consisté à mesurer la production du lait chez la vache laitière, en fonction de la présence ou de l'absence d'un additif alimentaire contenant un mélange de 28 % d'eugenol et de 17 % de cinnamaldehyde.

La ration alimentaire était à base de maïs, d'ensilage de maïs, de tourteaux de soja.

L'additif alimentaire a conduit à ajouter environ 400 milligrammes de mélange d'eugenol et de cinnamaldehyde, par animal et par ration alimentaire.

L'essai a été effectué pendant les douze premiers mois de lactation sur 173 vaches Holstein partagées en deux groupes.

Un premier groupe, correspondant au contrôle négatif, a reçu une ration alimentaire complète dépourvue d'additif alimentaire.

Un second groupe a reçu la même ration alimentaire supplémentée avec la combinaison d'eugenol et de cinnamaldehyde indiquée ci-dessus.

Les résultats obtenus sont indiqués dans le tableau 3 ci-dessous :

| Traitement | Groupe Contrôle (sans additif) | Groupe Expérimental (Eugenol + Cinnamaldehyde) |
|---|---|---|
| Nombre d'animaux | 97 | 76 |
| Consommation de Matière Sèche (kg MS/animal/jour) | 22.7 | 22.7 |
| Production du lait (kg/animal/jour) | 34.2 | 36.1 |
| Rendement protéique du lait (g/animal/jour) | 1259 | 1302 |
| Rendement butyreux du lait (g/animal/jour) | 983.4 | 1031 |

Le résultat montre une amélioration sensible de la production laitière par ajout d'un additif alimentaire selon l'invention.

### Etude 4

Cette quatrième étude a porté sur la mesure de la croissance de bovins à viande.

Cette étude a été effectuée pendant 75 jours dans des conditions classiques d'élevage en France sur des bovins à viande de la race Veaux Blonds d'Aquitaine. Le troupeau a été divisé en deux groupes de 15 animaux.

Un premier groupe (le groupe de contrôle négatif) a reçu une ration à base d'ensilage de maïs et de céréales, dépourvue d'additif.

L'autre groupe (le groupe expérimental) a reçu la même ration alimentaire mais supplémentée avec un mélange d'eugenol et de cinnamaldehyde selon l'invention, en proportion de 28 % d'eugenol et de 17 % de cinnamaldehyde. La dose était d'environ 400 milligrammes de mélange d'eugenol et de cinnamaldehyde par animal et par ration alimentaire.

Le poids vif des animaux était mesuré au début puis à 75 jours.

Les résultats sont exposés dans le tableau 4 ci-dessous :

| Traitement | Groupe Contrôle (sans additif) | Groupe Expérimental (Eugenol + Cinnamaldehyde) |
|---|---|---|
| Nombre d'animaux | 15 | 15 |
| Poids vif au début (kg/animal) | 218.5 | 219.6 |
| Poids vif à 75 jours (kg/animal) | 314 | 323 |
| Gain poids journalier (kg/animal/jour) | 1.27 | 1.39 |

Le résultat montre une augmentation de la vitesse de croissance en poids des bovins alimentés avec une ration contenant un additif alimentaire à base d'eugenol et de cinnamaldehyde selon l'invention.

### Etude 5

Cette cinquième étude a porté sur la recherche de la dose optimale de mélange eugenol-cinnamaldehyde dans la ration alimentaire des animaux ruminants. Il s'agit à nouveau d'une étude expérimentale "in vitro", dans laquelle le mélange eugenol-cinnamaldehyde a été incorporé dans certaines rations alimentaires mélangées comprenant de l'avoine fraîche, du foin de luzerne, des grains de blé, et du tournesol.

Les caractéristiques chimiques des rations alimentaires sont indiquées dans le tableau 5 ci-dessous.

| **Aliment** | **Matière sèche** | **Protéine totale** | **NDF** | **ADF** | **Amidon** |
|---|---|---|---|---|---|
| **Avoine** | 87,0 | ND* | 35,9 | 21,7 | ND |
| **Foin de luzerne** | 86,8 | ND | 30,8 | 25,4 | ND |
| **TMR** | 93,0 | 16,1 | 30,0 | 15,8 | 37,2 |
| * ND = non déterminé | | | | | |

Dans le tableau ci-dessus, les caractéristiques de chaque aliment sont données en pourcentage de matière sèche, de protéine totale, de fibre digestible neutre (NDF), de fibre digestible acide (ADF), pour l'avoine, la luzerne, une ration complète (TMR).

Le mélange eugenol-cinnamaldehyde a été préparé dans tous les essais dans la proportion 28 % d'eugenol et 17 % de cinnamaldehyde.

On a analysé les effets d'un mélange selon l'invention sur la digestibilité de la ration alimentaire selon la méthode de Tilley et Terry.

Le mélange a été inclus dans les rations alimentaires selon des proportions respectives de 3, de 30 et de 300 mg par jour et par animal, en utilisant des bovins qui consomment en moyenne 20 kg de matière sèche par jour.

Une comparaison a été faite avec des rations alimentaires contenant de la monensine de sodium dissoute dans l'éthanol, sans mélange eugenolcinnamaldehyde.

Des bouteilles de contrôle ont également été préparées, contenant de l'éthanol, pour éviter les effets parasites de la fermentation due à l'éthanol. Le fluide du rumen a été collecté à partir de deux bovins, sur lesquels on a adapté des canules, et qui ont été alimentés par une ration similaire de celle utilisée dans l'étude. Un prélèvement de 1 g de matière sèche a été pesé dans trois bouteilles de 125 ml de capacité, et trois bouteilles additionnelles ont été utilisées pour des contrôles négatifs contenant une solution tampon et un fluide ruminal seulement, pour la correction. 10 ml de fluide ruminal ont été ajoutés avec 40 ml de tampon anaérobique dans chaque bouteille. 0,5 g de chaque prélèvement a été pesé dans des filtres (de marque ANKOM), qui ont été alors incubés dans des flacons de 250 ml avec le fluide ruminal et le tampon pendant 24 heures, additionnés dans des mêmes proportions que pour des mesures de production de gaz.

Les résultats ont été analysés en utilisant les procédures mixtes SAS. On a comparé les résultats avec la ration contenant la monensine, en considérant que la ration avec la monensine donne un résultat de référence.

Les résultats obtenus sont contenus dans le tableau 6 ci-après qui reflète la digestibilité des rations alimentaires contenant des doses de mélange d'eugenol et de cinnamaldehyde de 3, 30 et 300 mg par animal et par jour (respectivement MIX 3, MIX 30, MIX 300) en comparant avec la digestibilité de la ration contenant la monensine (MON).

| **Traitement** | **DMd** | **NDFd** | **ADFd** |
|---|---|---|---|
| MON | 43,43 | 22,24 | 24,38 |
| MIX3 | 43,96 | 22,16 | 22,55 |
| MIX30 | 45,70 | 26,74 | 27,54 |
| MIX300 | 45,23 | 25,29 | 28,597 |

Ce tableau reflète ainsi la digestibilité de la fibre digestible neutre (NDFd), la digestibilité de la fibre digestible acide (ADFd), et la digestibilité de matière sèche (DMd), au bout de 24 heures.

Le tableau montre que les effets du mélange eugenol-cinnamaldehyde sur la digestibilité de la matière sèche et des fibres ne sont pas meilleurs que ceux de la monensine pour le niveau le plus bas, c'est-à-dire pour la dose de 3 mg par animal et par jour, qu'un maximum est obtenu pour la dose de 30 mg par jour et par animal, et qu'une amélioration est encore obtenue, quoique plus faible, pour une dose de 300 mg par animal et par jour.

On en déduit que la dose journalière de mélange d'eugenol et de cinnamaldehyde peut avantageusement être comprise entre 20 et 400 mg par jour et par animal pour une consommation de 20 kg de matière sèche. Cela correspond à une proportion de 1 à 20 ppm de mélange d'eugenol-cinnamaldehyde par rapport à la matière sèche des rations.

A partir des études effectuées, on a pu déterminer qu'une amélioration de la digestibilité des aliments est obtenue par l'action d'un mélange d'eugenol et de cinnamaldehyde, dans lequel le rapport entre les quantités respectives en poids d'eugenol et de cinnamaldehyde est supérieur à 1 et inférieur à 5.

Il apparaît que des résultats plus avantageux sont obtenus par un mélange dans lequel le rapport entre les quantités respectives en poids d'eugenol et de cinnamaldehyde est compris entre 1 et 1,8 environ.

Le mélange d'eugenol et de cinnamaldehyde peut être utilisé pour réaliser un additif alimentaire. Dans l'additif alimentaire, le mélange d'eugenol et de cinnamaldehyde peut avantageusement être présent en proportion comprise entre 0,00005 % et 5 % en poids. L'excipient formant l'additif alimentaire avec le mélange ci-dessus peut contenir, en poids, 0,1 % à 1 % d'un mélange de vitamines, de 20 % à 80 % de sels minéraux, de 20 % à 80 % de protéines, de 20 % à 80 % de produits issus de meunerie.

Selon une autre application, le mélange peut être utilisé pour réaliser une pierre à lécher. Dans ce cas, le mélange peut être associé à un ou des sels minéraux pris dans la famille des sels de phosphore, de calcium, de magnésium.

Le mélange peut également être utilisé pour réaliser des rations pour animaux ruminants.

Une telle ration peut par exemple contenir :
- le mélange eugenol-cinnamaldehyde défini ci-dessus,
- au moins 50 % de céréales,
- au moins 25 % de tourteaux d'oléagineux,
- au moins 20 % de produits issus de meunerie.

La présente invention n'est pas limitée aux modes de réalisation qui ont été explicitement décrits, mais elle en inclut les diverses variantes et généralisations contenues dans le domaine des revendications ci-après.

## Revendications

1. Utilisation d'un mélange contenant de l'eugenol et du cinnamaldehyde pour améliorer la digestibilité des aliments par les animaux ruminants, **caractérisée en ce que** le rapport entre les quantités respectives en poids d'eugenol et de cinnamaldehyde est compris entre 1 et 5, et **en ce que** le mélange d'eugenol et de cinnamaldehyde est adsorbé ou absorbé sur un support solide particulaire.

2. Utilisation d'un mélange contenant de l'eugenol et du cinnamaldehyde pour améliorer la digestibilité des aliments par les animaux ruminants, **caractérisée en ce que** le rapport entre les quantités respectives en poids d'eugenol et de cinnamaldehyde est compris entre 1 et 5, **en ce que** le mélange d'eugenol et de cinnamaldehyde est incorporé à la ration alimentaire de l'animal, et **en ce que** la ration alimentaire contient le mélange d'eugenol et de cinnamaldehyde avec :
- au moins 50 % de céréales,
- au moins 25 % de tourteaux d'oléagineux,
- au moins 20 % de produits issus de meunerie.

3. Procédé pour augmenter l'efficacité de l'utilisation des aliments par un animal ruminant, à savoir pour augmenter la digestibilité des aliments par l'animal ruminant, ou pour augmenter la production laitière de l'animal ruminant, ou pour augmenter la vitesse de croissance en poids de l'animal ruminant, comprenant le fait d'ajouter à la ration alimentaire de l'animal un mélange d'eugenol et de cinnamaldehyde en quantité appropriée pour augmenter la digestibilité de la ration alimentaire, **caractérisé en ce que** le rapport entre les quantités respectives en poids d'eugenol et de cinnamaldehyde est compris entre 1 et 5, et **en ce que** le mélange d'eugenol et de cinnamaldehyde est adsorbé ou absorbé sur un support solide particulaire, puis le support solide particulaire contenant le mélange est ajouté à la ration alimentaire.

4. Additif alimentaire pour les animaux ruminants, comprenant un mélange d'eugenol et de cinnamaldehyde adsorbé ou absorbé sur un support solide particulaire, **caractérisé en ce que** le rapport entre les quantités respectives en poids d'eugenol et de cinnamaldehyde est compris entre 1 et 5, et **en ce qu'**il contient un excipient contenant, en poids, de 0,1 % à 1 % d'un mélange de vitamines, de 20 % à 80 % de sels minéraux, de 20 % à 80 % de protéines, de 20 % à 80 % de produits issus de meunerie.

5. Utilisation selon la revendication 2, **caractérisée en ce que** le mélange d'eugenol et de cinnamaldehyde est présent en proportion de 1 ppm à 20 ppm environ par rapport à la matière sèche des rations alimentaires du ruminant.

6. Procédé selon la revendication 3, **caractérisé en ce que** la proportion du mélange d'eugenol et de cinnamaldehyde est de 1 ppm à 20 ppm environ par rapport à la matière sèche des rations alimentaires de l'animal.

## Patentansprüche

1. Verwendung einer Eugenol und Zimtsäurealdehyd enthaltenden Mischung zur Verbesserung der Nahrungsmittelverdaulichkeit bei Wiederkäuern, **dadurch gekennzeichnet, dass** das Verhältnis zwischen den Gewichtsanteilen von Eugenol und Zimtsäurealdehyd zwischen 1 und 5 liegt, **und dadurch, dass** die Mischung aus Eugenol und Zimtsäurealdehyd auf einem teilchenförmigen Feststoffträger adsorbiert oder absorbiert ist.

2. Verwendung einer Eugenol und Zimtsäurealdehyd enthaltenden Mischung zur Verbesserung der Nahrungsmittelverdaulichkeit bei Wiederkäuern, **dadurch gekennzeichnet, dass** das Verhältnis zwischen den Gewichtsanteilen von Eugenol und Zimtsäurealdehyd zwischen 1 und 5 liegt, **und dadurch, dass** die Mischung aus Eugenol und Zimtsäurealdehyd der Nahrungsration des Tieres beigemengt ist, **und dadurch, dass** die Nahrungsration die Mischung aus Eugenol und Zimtsäurealdehyd und:
- wenigstens 50% Getreide,
- wenigstens 25% Ölkuchen,
- wenigstens 20% Mühlenprodukte
enthält.

3. Verfahren zum Erhöhen der Wirksamkeit bei der Verwendung der Nahrungsmittel für einen Wiederkäuer, und zwar um die Nahrungsmittelverdaulichkeit bei Wiederkäuern zu erhöhen, oder um die Milchproduktion bei Wiederkäuern zu erhöhen, oder um die Geschwindigkeit der Gewichtszunahme zu erhöhen, umfassend, dass der Nahrungsration für das Tier eine Mischung aus Eugenol und Zimtsäurealdehyd in einer Menge hinzugefügt wird, die geeignet ist, die Verdaulichkeit der Nahrungsration zu steigern, **dadurch gekennzeichnet, dass** das Verhältnis zwischen den Gewichtsanteilen von Eugenol und Zimtsäurealdehyd zwischen 1 und 5 liegt **und dadurch, dass** die Mischung aus Eugenol und Zimtsäurealdehyd auf einem teilchenförmigen Feststoffträger adsorbiert oder absorbiert wird und dann der die Mischung enthaltende teilchenförmige Feststoffträger der Nahrungsration beigegeben wird.

4. Nahrungsmittelzusatzstoff für Wiederkäuer, umfassend eine Mischung aus Eugenol und Zimtsäurealdehyd, die auf einem teilchenförmigen Feststoffträger adsorbiert oder absorbiert ist, **dadurch gekennzeichnet, dass** das Verhältnis zwischen den Gewichtsanteilen von Eugenol und Zimtsäurealdehyd zwischen 1 und 5 liegt **und dadurch, dass** dieser ein Excipient enthält, das in Gewichtsprozent zwischen 0,1% und 1% einer Mischung aus Vitaminen, zwischen 20% und 80% Mineralsalze, zwischen 20% und 80% Proteine und zwischen 20% bis 80% Mühlenprodukte enthält.

5. Verwendung nach Anspruch 2, **dadurch gekennzeichnet, dass** die Mischung aus Eugenol und Zimtsäurealdehyd im Verhältnis von etwa 1 ppm bis 20 ppm bezogen auf die Trockensubstanz der Nahrungsrationen des Wiederkäuers in dieser vorhanden ist.

6. Verfahren nach Anspruch 3, **dadurch gekennzeichnet, dass** das Verhältnis der Mischung aus Eugenol und Zimtsäurealdehyd bezogen auf die Trockensubstanz der Nahrungsrationen des Wiederkäuers etwa 1 ppm bis ca. 20 ppm beträgt.

## Claims

1. Use of a mixture containing eugenol and cinnamaldehyde to improve the digestibility of food by the ruminant animals, **characterized in that** the ratio between the respective quantities by weight of eugenol and cinnamaldehyde lies between 1 and 5, and **in that** the eugenol and cinnamaldehyde mixture is adsorbed or absorbed on a solid particulate substrate.

2. Use of a mixture containing eugenol and cinnamaldehyde to improve the digestibility of food by the ruminant animals, **characterized in that** the ratio between the respective quantities by weight of eugenol and cinnamaldehyde lies between 1 and 5, **in that** the eugenol and cinnamaldehyde mixture is incorporated in the animal feed, and **in that** the feed contains the eugenol and cinnamaldehyde mixture with:
- at least 50% cereals,
- at least 25% oil-cakes,
- at least 20% flour mill bi-products.

3. Method for increasing the effectiveness of food use by a ruminant animal, namely to improve the digestibility of feed by the ruminant animal, or to improve milk production by the ruminant animal, or to improve the growth rate by weight of the ruminant animal, by adding to the feed of the animal a mixture of eugenol and cinnamaldehyde in a suitable quantity to increase the digestibility of the feed, **characterized in that** the ratio between the respective quantities by weight of eugenol and cinnamaldehyde lies between 1 and 5, and **in that** the eugenol and cinnamaldehyde mixture is adsorbed or absorbed on a solid particulate substrate, then the solid particulate substrate containing the mixture is added to the feed.

4. Food additive for ruminant animals, including a mixture of eugenol and cinnamaldehyde adsorbed or absorbed on a solid particulate substrate, **characterized in that** the ratio between the respective quantities by weight of eugenol and cinnamaldehyde lies between 1 and 5, and **in that** said food additive contains a carrier containing by weight a vitamin mixture at between 0.1% and 1%, mineral salts at between 20% and 80%, proteins at between 20% and 80%, and flour mill bi-products at between 20% and 80%.

5. Use according to claim 2, **characterized in that** the mixture of eugenol and cinnamaldehyde is present in a ratio of approximately 1 ppm to 20 ppm as compared to the dry matter of the feed for ruminant animals.

6. Method according to claim 3, **characterized in that** the mixture of eugenol and cinnamaldehyde is present in a ratio of approximately 1 ppm to 20 ppm as compared to the dry matter of the feed for ruminant animals.
